# EUROPEAN PATENT SPECIFICATION

(11) **EP 4 017 448 B1**
(45) Date of publication and mention of the grant of the patent: **04.10.2023**
(21) Application number: 20760933.0
(22) Date of filing: 17.08.2020
(51) Int. Cl.: A61F 13/00, C12Q 1/24, A61F 13/38, D04H 1/4374

(54) **ARTICLE USEABLE FOR SAMPLING SURFACES FOR MICROORGANISMS AND METHODS OF USE**
ARTIKEL FÜR PROBENNAHME VON OBERFLÄCHEN FÜR MIKROORGANISMEN UND VERWENDUNGSVERFAHREN
ARTICLE POUVANT ÊTRE UTILISÉ POUR ÉCHANTILLONNER DES SURFACES POUR DES MICROORGANISMES ET PROCÉDÉS D'UTILISATION

(30) Priority: 21.08.2019 US 201962889702 P
(43) Date of publication of application: 29.06.2022
(73) Proprietor: Garden US Holdco Corporation, Lansing MI 48912 (US)
(72) Inventor: CHANDRAPATI, Sailaja, Saint Paul, Minnesota 55133-3427 (US); BATRA, Saurabh, Saint Paul, Minnesota 55133-3427 (US); SANOCKI, Stephen M., Saint Paul, Minnesota 55133-3427 (US); PARTLO, Walter E., Inver Grove Heights, Minnesota 55076 (US); HIBBARD, Lou D., Saint Paul, Minnesota 55133-3427 (US); DUNBAR, Joseph A., Saint Paul, Minnesota 55133-3427 (US)
(74) Representative: Mathys & Squire
(86) International application number: PCT/IB2020/057748
(87) International publication number: WO 2021/033130

(56) References cited:
- US-A1- 2003 217 516

## Description

### TECHNICAL FIELD

The present disclosure relates to articles suitable for and methods of collecting a sample of microorganisms from a surface.

### BACKGROUND

WO2015123635, USD809234, and US20170051442 describe scouring articles. Such articles are not necessarily useful or convenient for collecting samples of microorganism; US2003/217516 discloses a nonwoven composite cleaning pad.

### SUMMARY

In one aspect of the present invention there is provided an article as set out in claim 1. In another aspect, there is provided a method of collecting a sample of microorganisms from a surface, the method comprising contacting a surface with the article of the present disclosure.

### BRIEF DESCRIPTION OF THE DRAWINGS

Figure 1 is a cut-away side view of a portion of a sampling article;
Figure 2 is a cut-away side view of a portion of a sampling article;
Figure 3 is a top-down view of a sampling article; and
Figure 4 is a side view of a sampling article.

### DETAILED DESCRIPTION

Throughout this disclosure, singular forms such as "a," "an," and "the" are often used for convenience; however, it should be understood that the singular forms are meant to include the plural unless the singular alone is explicitly specified, such as by terms such as "one and only one," or is clearly indicated by the context.

A layer disposed over "a portion of" a second layer is disposed over part, but less than all, of the second layer. Typically, a layer disposed over a portion of a second layer is disposed over at least 1%, 5%, 10%, 20%, 30%, 40%, 50%, 60%, 70%, 80%, or in some cases even 90% of the second layer, but most commonly is disposed over no more than 70%, 60% 50%, 40%, 30%, 20%, or even 10% of the second layer. Most often, a layer disposed over a portion of a second layer is disposed over 20%-60% of the second layer.

A layer disposed over "at least a portion of' a second layer may be disposed over a portion of the second layer or over the entire second layer.

Unless specifically used with reference to the prior art, terms such as "common," "typical," and "often" are used to refer to features or embodiments that are commonly employed in the present disclosure. Use of such terms is not intended to suggest that the feature or embodiment being discussed was previously known, much less that it was commonly known.

The "solids content" of a nonwoven refers to the percent of solids, on a mass per volume basis, in the nonwoven. Solids content can be particularly calculated as the basis weight of the nonwoven divided by the product of the thickness of the nonwoven and the density of the solid, usually polymer, used to make the nonwoven, normalized to 100%. This can be expressed as m/(p*L), wherein m is the basis weight with units of mass per area (such as grams per meter squared), p is the thickness with units of distance (such as meters), and L is the density of the solid, usually polymer, in the nonwoven, in units of mass per volume (such as grans per meters cubed). The result is dimensionless value that can be expressed as a percent. Solids content is sometimes referred to in the art as "solidity"; however, when the terms solids content and solidity are used in the art they may not be used in the same sense as the term "solids content" is used here.

A problem in sampling devices relates to removing the sample from the device. Once a sample, such as an aqueous sample, is obtained, it can be difficult to release the sample from the sampling device, which typically contains a sponge or sponge-like material. It would be preferable to desorb the sample without the need for excessive wringing or manipulation of the sample device.

Sampling surfaces, such as environmental surfaces, drains, workstations, machine surfaces, food preparation surfaces, or even the surfaces of organisms (living or dead), for detecting microorganism on such surfaces can be important in order to ascertain, for example, whether the surface is contaminated with microorganisms. Such contamination can lead to food spoilage, illness, and other problematic consequences. Present sampling articles and methods can give rise to inaccurate results or even false negatives, particularly when food buildup or biofilms are present on the surface being sampled. Such inaccuracies or false negatives can manifest in that when the samples obtained by present sampling devices analyzed for the presence of microorganisms, for example by culturing the sample for particular microorganisms, no microorganisms are detected even though the microorganisms being cultured for are present on the surface that was sampled.

This disclosure recognizes two mechanisms giving rise to such inaccuracies or false negatives that relate to the sampling device. First, the sampling device may fail to pick up microorganisms from the surface being sampled. This is particularly problematic in the presence of microbes or samples that are difficult to dislodge from the surface that is sampled, such as biofilms. Biofilms and other samples that are difficult to dislodge can resist being transferred from the surface that is sampled to a sampling device. Thus, it is possible to incorrectly conclude that no microorganisms are present on the surface that is sampled when in fact the microorganisms are present but did not transfer to the sampling device. This incorrect conclusion is a type of false negative.

Second, even when microbes are transferred from the surface being sampled to the sampling device, the sampling device itself may kill some or all of the microbes thereby skewing the results or giving a false negative. For example, if an abrasive is used to break up the biofilm before sampling, the abrasive can disrupt the microorganism cell membrane and kill the microorganism. Also, if a biocide is present in the sampling device the biocide can impact recovery of microbes, particularly low-level microbial bioburden, because of inherent residual kill. In particular, sponges which are often used to collect samples in sampling devises, are typically made in the presence of water (known as a "wet environment") and therefore require the addition of biocides to prevent microbes from growing on the sponge. Thus, the inventors have recognized that sponges and other sampling devices with residual biocides or harsh abrasives in the sampling device can adversely impact the integrity of the sample even if the microbes are transferred from the surface to the sampling device. This is particularly problematic when it is desirable to culture the microorganism because microorganisms that were killed during the collection process will not grow to form colony units.

Another problem to be solved is how to avoid or at least reduce the occurrence of false negatives that are caused by the sampling device. Another problem is how to effectively dislodge and pick up samples, particularly hard to dislodge samples such as biofilms, from surfaces without killing the microbes, disrupting the cell membranes of the microorganisms, or otherwise adversely impacting sample integrity.

Briefly, a solution to one or more of these problems lies in article comprising an inner layer contained within one or more outer layers, the inner layer comprising a hydrophilic, absorbent textile, which can be woven, non-woven, or knit but is most often non-woven, having a solids content of 0.5% -30%, more particularly 0.5% to 15%, and the one or more outer layers comprising a porous, hydrophilic, wicking nonwoven, which can be woven, non-woven, or knit but is most often non-woven, having a solids content of 2% - 40%. A scouring layer is disposed over a portion of at least one of the one or more outer layers. The article further comprises one or more second portions of the one or more outer layers, wherein the scouring layer is not disposed over the one or more second portions.

In a particular, at least one of the one or more outer layers is a non-woven. In a particular case, the inner layer is a non-woven. In a particular case, one or more outer layers a non-woven and the inner layer is woven. In a particular case, one or more outer layers non-woven the inner layer is knit. In a particular case, one or more outer layers is woven, and the inner layer is a non-woven. In a particular case, one or more outer layers is woven, and the inner layer is woven. In a particular case, one or more outer layers non-woven and the inner layer is a non-woven. In the most particular case, which is the most common, all of the outer layers are non-woven and the inner layer is a non-woven.

Regardless of the nature of the textile, the inner layer is made of a hydrophilic wicking material, which can be any hydrophilic wicking textile, usually a non-woven, that can absorb aqueous samples, and particularly aqueous samples having one or more microorganisms. Any suitable hydrophilic fiber material can be used, for example rayon, wood pulp fibers, acrylic fibers nylon fibers, wool, cotton, nylon, polybutylene terephthalate (sometimes referred to in the art as PBT), hydrophilized polyolefin, such as hydrophilized polypropylene, hydrophilized polyethylene, or hydrophilized olefin copolymer, and gauzes. Fibers that are inherently hydrophobic can be used, in which case they can be finished with a hydrophilic coating. Most commonly the hydrophilic wicking material comprises viscose fibers, rayon fibers, viscose/PET (polyethylene terephthalate) fibers, rayon/PET fibers, or a combination of any of the foregoing. When the fibers are viscose/PET or rayon/PET, the PET content is typically less than 70%, more commonly less than 60%, 50%, 40%, 30%, 20%, or even less than 10%. When there is PET employed, a typical PET content is around 40%-60%%, though other contents may be used depending on the application and precise properties that are desired. When PET employed, it is often a coating on at least a portion of the fibers. In particular cases, PET is thermally bonded to the fibers.

The solids content of the inner layer is about 0.5%-15%. Particular solids contents are 0.5% or greater, 2% or greater, 5% or greater, 7% or greater, greater than 10%, or even 12% or greater. Particular solids contents are 15% or less, 12% or less, 10% or less, 7% or less, 5% or less, 2% or less, or even 1% or less. Materials with appropriate solids content can be manufactured by any suitable methods, including carding, spunbonding, spunlacing, melt blowing, air laying, creped, and the so like. Some suitable processes are described in US7393371. Materials usable for the inner layer are also commercially available, and include gauze, the high loft nonwoven available under the trade designation Reicofil from Reifenhauser Reicofil GmnH & Co. KG (Troisdorf, Germany), and high loft nonwovens available under the trade designation SURELIFT from Vita Nonwovens (High Point, NC, USA).

The solids content of the material in the inner layer is critical to provide the correct properties. If the solids content is too high, then it will be difficult to release all of the sample that is absorbed in the inner layer. If the solids content is too low, the inner layer will have insufficient structural integrity to remain integral when wet.

Notably, while the inner layer can be made of cellulose-containing fibers, the inner layer is not a sponge, and particularly not a cellulose sponge. Indeed, nonwovens, such as the nonwovens used for the inner layer and the outer layer of the article described herein, are recognized as being structurally distinct from sponges and have different physical properties from those of sponges. The use of sponges as inner layers is not suitable for this invention because sponges do not have appropriate release properties to easily, and preferably reproducibly, release the sample that is absorbed.

The one or more outer layers comprising a porous, hydrophilic, wicking material, which is usually in the form of a textile but can also be a membrane in particular cases. When a textile, it can be a non-woven, woven, or knit. The one or more outer layers have a solids content of about 2%-40%. Particular values can be 2% or greater, 5% or greater, 10% or greater, 15% or greater, 20% or greater, 25% or greater, 30% or greater, or even 35% or greater. Particular values can be 40% or less, 35% or less, 30% or less, 25% or less, 20% or less, 15% or less, or even 10% or less.

The solids content of the one or more outer layers is also critical. If the solids content is too low, then the one or more outer layers will either not be sufficiently integral to contain the inner layer when the inner layer is wet, or the one or more outer layers will absorb too much water and the release properties of the overall device will not be adequate. If the solids content is too high, then the one or more outer layers may not pick up microorganisms from the surface being sampled.

The pore size of the one or more outer layers can be sufficient to allow a microorganism to pass through the pores, for example, from 20 nm to 5 microns. Any suitable hydrophilic fiber material can be used, for example rayon, wood pulp fibers, acrylic fibers, nylon fibers, wool, cotton, and fibers having hydrophilic coatings for finishes, but most commonly the hydrophilic wicking non-woven employs viscose fibers, rayon fibers, viscose/PET (polyethylene terephthalate) fibers, rayon/PET fibers, or a combination of any of the foregoing. When the fibers are viscose/PET or rayon/PET, the PET content is typically less than 70%, more commonly less than 60%, 50%, 40%, 30%, 20%, or even less than 10%. When PET is employed, a typical PET content is about 15-35%, though other contents may be used depending on the application and precise properties that are desired. When PET employed, it is can be, for example, thermally bonded to the fibers or a coating on at least a portion of the fibers.

Materials with appropriate solids content and pore size for use in the one or more outer layers can be manufactured by any suitable methods, such as those described in US7393371. Materials usable for the outer layer are also commercially available and include those available under the trade designation SX567, Grade 200 50/50 rayon/polyester spunlace, and SX632, all of which are available from Alstrom Corp. (Green Bay, WI, USA), as well as those available under the trade designation SONTARA, particularly SONTARA 8010, and available from DuPont Nonwovens Inc. (Old Hickory, TN, USA).

The inner layer and the one or more outer layers are in most cases free of biocides. In some cases, which are possible but rarely employed, residual biocides may be present, but such residual biocides are insufficient to kill microbes on the sampling device, or insufficient, either because of the type of biocide or because of the concentration of biocide, to kill the type or types of microbes that are to be detected. It is however possible for biocides to be present in or on the handle of the article, if a handle is used, or in or on other parts of the article which do not contact the surface or microbes to be sampled.

The article can be made by any suitable process. The various layers of the article can be attached by any suitable bonding process, such as thermal bonding, adhesive bonding, hydroentangling, needle-punching, calendaring, and heat bonding, such as point bonding. When a handle is used, these same techniques can be used to affix the

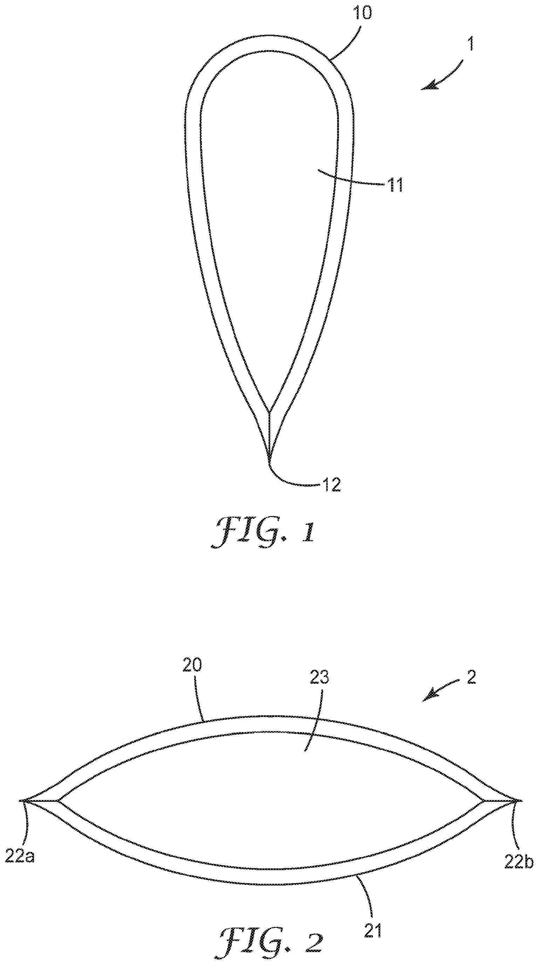

While a variety of configurations of the inner layer and the one or more outer layers are possible, two will be discussed in detail here. The first configuration is shown in Figure 1, wherein article 1 contains outer layer **10** that is sealed at seal **12.** Inner layer **11** is completely contained within outer layer **10.**

The second primary configuration is shown in Figure 2. In this configuration, article 2 has first outer layer **20** and second outer layer **22,** which are sealed together at seals **22a** and **22b** such that inner layer **23** is completely contained within the first and second outer layers **20, 21.**

In any configurations, the seals can be formed in any suitable manner depending on the intended use of the article and the precise content of the inner and outer layers. Exemplary seals can be formed by use of adhesive or by heat-sealing.

Other configurations are also possible. For example, it is possible to seal together three, four, or even more outer layers to contain an inner layer as described above, in which case a cross-section of the article's inner and outer layers might be approximately triangular, square, or some other shape. Such configurations are encompassed by the present disclosure; because numerous additional configurations are readily envisioned based on this disclosure, every possible configuration will not be described in detail.

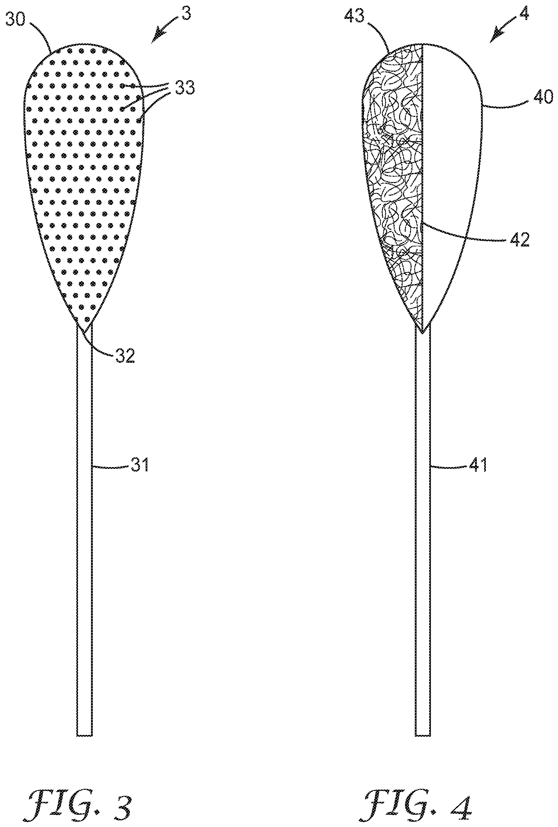

It is often convenient to include a handle, such as handle **31,** which is visible in Figure 3. When a handle is included, the one or more outer layers, such as outer layer **32** in Figure 3, will typically be sealed to the handle in addition to being sealed to themselves.

The scouring layer, which is present on a portion of the one or more outer layers regardless of the configuration of the inner and outer layers, is typically a cured resin. Optionally, the resin can have a plurality of abrasive particles embedded or otherwise within the resin. When employed, the abrasive particles are typically mildly abrasive. The abrasive particles, if employed, typically have a Mohs hardness of at least 3. Glass beads can be used.

Abrasive particles are not required in all cases. In fact, in some situations, such as when the end user is concerned about the potential to scratch a surface that is being sampled, then it can be advantageous to employ a scouring layer does not include abrasive particles.

In principle, and irrespective of whether abrasive particles are employed, any resin can be used. Particular resins that can be employed include those that have very mild abrasive properties, for example, so that they can avoid scratching or damaging the surfaces being sampled. Cured phenolic resins are common because they are convenient to manufacture and place on the one or more outer layers, but other resins can also be used. Abrasives and abrasive particles that can be employed include those described in US7393371 and WO2015123635. Scouring layers that can be employed, even without abrasive particles, include those disclosed in WO2015123635.

The scouring layer can be disposed in different ways over a portion of the one or more outer layers. The scouring layer can be continuous or discontinuous. When discontinuous, the scouring layer is commonly disposed as a repeating pattern of one or more geometric shapes, such as circles, squares, lines, "saw-tooth" or "squiggly" lines, triangles, or rhombuses. An example of this configuration is shown in Figure 3, which features article **3** having outer layer **30** and handle **31.** Outer layer **30** is sealed to itself and to handle **31** at seal **32.** The inner layer, which is not visible in this Figure, is disposed within outer layer **30** in a similar manner to inner layer **11** as depicted in Figure 1. The discontinuous scouring layer is present as a plurality of circles 33 that are arranged in a repeating pattern on outer layer **30.** It is possible for a discontinuous scouring layer to be on the entire outer layer of an article, such as article **3,** or on only a portion of the outer layer of an article.

The scouring layer can also be continuous. An example of a continuous scouring layer is scouring layer **43** of article **4** as depicted in Figure 4. In Figure 4, there are two portions to outer layer **40.** The first portion is not visible in the Figure because it is completely covered by continuous scouring layer **43.** The second portion of outer layer **40,** which is on the opposite side of handle **41** from the first portion, is not covered by scouring layer **43.** The two portions are sealed to each other and to handle **41** by way of seal **42.**

In use, a method of collecting microorganisms from a surface can comprise contacting microorganisms on a surface with the at least a portion of the scouring layer of an article as described herein and subsequently contacting the microorganisms with at least a portion of an outer layer of an article as described herein. In most cases, contacting with the scouring layer will include scouring the surface with the scouring layer.

Typically, either the article, and particularly its one or more layers, or the surface will be moist during the process. Moistening is typically with water or an aqueous liquid. Buffered water is an exemplary aqueous liquid.

The method can be carried out on nearly any surface. Common surfaces to which the method can be applied include food preparation surfaces, workstation surfaces, or machine surfaces, such as cutting, grinding, or sorting machines used in food preparation. Other surfaces that can be sampled include walls, and drains.

The method can also be applied to biological surfaces, such as a biofilm-coated surface, a skin surface, or a wound surface. When a biological surface is used, the method can be useful, for example, to collect microorganisms that may be present on a food substance's surface or to collect microorganisms that may be present on a surface of a living organisms for the ultimate purpose of identifying or enumerating the microorganisms present on the surface of the living organism.

The method can also include the step of releasing the microorganisms that were collected from the article, and typically into a liquid. This can be accomplished, for example, by contacting the article with the liquid. The liquid can be any suitable liquid, such as a lysis buffer, growth media, selective growth media, or enrichment media. The microorganisms can also be released into a gel, such as a gelled media.

The method can further include additional steps such as culturing the microorganisms, identifying the microorganisms, enumerating the microorganisms, or a combination of one or more of the foregoing steps.

### EXAMPLES

These examples illustrate certain specific embodiments or implementations of the disclosure. Other embodiment and implementations are also contemplated, so these are not intended to limit the appended claims which may include both these examples as well as other embodiments or implementations.

### Materials

BB-077 Phenolic resin (70% aqueous), commercially available from Arclin, Mississauga, Ontario. SNOWHITE 12-PT filler, a calcium carbonate powder (density of 2.7 cm³), commercially available from Omya International AG, Oftringen, Switzerland.

MAXI-CLEAN 100 mesh, hardened, melamine resin granules (4.0 Mohs hardness), commercially available from Maxi-Blast Inc., South Bend, IN.

MAXI-CLEAN 200 mesh, hardened, melamine resin granules (4.0 Mohs hardness), commercially available from Maxi-Blast Inc., South Bend, IN.

XIAMETER AFE-1010 anti-foam emulsion, commercially available from Dow Chemical, Midland, MI.

SUNSPERSE Blue (BCD 9680) blue pigment, commercially available from the Sun Chemical Corporation, Cincinnati, OH.

ARCOL LG-650 polyether polyol cross linker, commercially available from Covestro, Pittsburgh, PA.

CAB-O-SIL M-5 filler, an untreated, amorphous, synthetic fumed silica with a specific surface area of 200 m²/g, commercially available from the Cabot Corporation, Billerica, MA.

ROVENE 5900 latex, a carboxylated styrene-butadiene emulsion with a Brookfield viscosity of 200 cps (#2 spindle/20 rpm) and pH of 9.0, commercially available from Mallard Creek Polymers Incorporated, Charlotte, NC.

XIAMETER AFE-1520 silicone emulsion (density of 1.0 g/mL and pH of 4), commercially available from the Dow Corning Corporation, Midland, MI.

SLTNSPERSE (RPD 2542) liquid red pigment, commercially available from the Sun Chemical Corporation, Cincinnati, OH.

INDOLUBE PPL surfactant, a nonionic stabilizer for latex emulsions (density of 1.02 g/mL), commercially available from Indusco, Ltd., Greensboro, NC.

LYCOPRINT PT-XN thickener, a fully neutralized, anionic acrylic polymer dispersion (density of 1.1 g/mL), commercially available from Huntsman International LLC, High Point, NC.

**Table 1. Resin Formulation A for Scouring Layer**

| Component | Weight Percent |
|---|---|
| BB-077 Phenolic Resin (70% aqueous) | 70.65 |
| SNOWHITE 12-PT calcium carbonate filler | 10 |
| MAXI-CLEAN 100 mesh melamine resin granules | 11.2 |
| MAXI-CLEAN 200 mesh melamine resin granules | 2.8 |
| XIAMETER AFE-1010 anti-foam emulsion | 0.1 |
| SUNSPERSE Blue (BCD 9680) blue pigment | 0.25 |
| ARCOL LG-650 polyether polyol cross linker | 2 |
| CAB-O-SIL M-5 filler | 1.5 |
| Water | 1.5 |

The components of Resin Formulation A (Table 1) were combined as a 1000 g batch in a rigid plastic container and a plastic lid was attached to the container. The mixture was mixed in a centrifugal mixer (model 'SPEEDMIXER DAC 400.1 VAC-P' that was obtained from Flaktek, Inc., Landrum, SC) for about 60 minutes.

**Table 2. Resin Formulation B for Scouring Layer**

| Component | Weight Percent |
|---|---|
| ROVENE 5900 latex emulsion | 65.00 |
| XIAMETER AFE-1520 silicone emulsion | 0.10 |
| SUNSPERSE (RPD 2542) red pigment | 0.85 |
| INDOLUBE PPL surfactant | 4.50 |
| SNOWHITE 12-PT calcium carbonate filler | 28.92 |
| LYCOPRINT PT-XN thickener | 0.63 |

The components of Resin Formulation B (Table 2) were combined as a 1000 g batch in a rigid plastic container and a plastic lid was attached to the container. The mixture was mixed in a centrifugal mixer (model 'SPEEDMIXER DAC 400.1 VAC-P' that was obtained from Flaktek, Inc., Landrum, SC) for about 60 minutes.

### Example 1.

A three-layer sampling article was prepared having an inner layer and two outer layers. The nonwoven web first outer layer [SONTARA brand (material no. 3006871) obtained from the Jacob Holm Group, Basel, Switzerland] was a blend of PET fibers (60 % by weight) and viscose fibers (40% by weight). The density of the PET fiber was about 1.38 g/cm³ and the density of the viscose fiber was about 1.49 g/cm³. The nominal basis weight of the web was 43 grams per square meter (gsm) and the web thickness was about 0.8 mm. The solids content of the nonwoven web was about 3.8%.

The nonwoven web second outer layer [SONTARA brand (material no. 3006871) obtained from the Jacob Holm Group] was a blend of PET fibers (60% by weight) and viscose fibers (40% by weight). The density of the PET fiber was about 1.38 g/cm³ and the density of the viscose fiber was about 1.49 g/cm³. The nominal basis weight of the web was 43 grams per square meter (gsm) and the web thickness was about 0.8 mm. The solids content of the nonwoven web was about 3.8%. The scouring layer was applied in a dot pattern to one surface of the second outer layer using a rotary screen printing apparatus and a slurry of Resin Formulation A (described above). A stencil was used to create the repeating dot pattern. The stencil had holes that were 2.5 mm in diameter with a center-to-center spacing of 7.5 mm in offset rows. The resulting printed web was passed through a heating apparatus (set at 1.7 meters per minute and 160 °C) to solidify the dots of resin.

The nonwoven web inner layer was prepared using a conventional air-laying web forming machine (available from the Rando Machine Company, Macedon, NY under the trade designation RANDO WEBBER). The web was a blend of viscose fibers (52% by weight), PET fibers (18% by weight), and melty fibers (30% by weight). The viscose fiber (diameter of 10-18 microns) was obtained as LENZING viscose fiber from the Lenzing Group (Lenzing, Austria) and had a fiber length of 3.9 cm. The PET fiber (3 denier) was obtained from the INVISTA Company (Wichita, KS) and had a fiber length of 3.8 cm. The melty fiber (4 denier) was Tairilin polyester Type LML41 (NanYa Plastics Corporation, Kaohsiung City, Taiwan) and had a fiber length of 5 cm. The resulting nonwoven web had a nominal basis weight of 222 grams per square meter (gsm) and a web thickness of 5.2 mm. The solids content of the nonwoven web was about 2.9%. The web was needle tacked and thermally bonded by passing the web through a heating apparatus (set at 1.1 meters per minute and 138° C).

Each nonwoven layer was cut into 1.5 inch by 3 inch (3.8 cm by 7.6 cm) sections. The layers were stacked and edge aligned so that one surface of the inner nonwoven layer was in contact with the first outer layer and the opposite surface of the inner layer was in contact with the second outer layer. The second outer layer was oriented so that the scouring layer was exposed (i.e. facing away from the inner layer). The edges were heat sealed at 232° C to form the finished sampling article.

### Example 2.

A three layer sampling article was prepared having an inner layer and two outer layers. The nonwoven web first outer layer [SONTARA brand (material no. 444515177) obtained from the Jacob Holm Group, Basel, Switzerland] was a blend of PET fibers (45% by weight) and wood pulp (55% by weight). The density of the PET fiber was about 1.38 g/cm³. The nominal basis weight of the web was 65 grams per square meter (gsm) and the web thickness was about 0.26 mm. The solids content of the nonwoven web was about 17.9%.

The nonwoven web second outer layer [SONTARA brand (material no. 444515177) obtained from the Jacob Holm Group] was a blend of PET fibers (45% by weight) and wood pulp (55% by weight). The density of the PET fiber was about 1.38 g/cm³. The nominal basis weight of the web was 65 grams per square meter (gsm) and the web thickness was about 0.26 mm. The solids content of the nonwoven web was about 17.9 %. The scouring layer was applied in a dot pattern to one surface of the second outer layer using a rotary screen printing apparatus and a slurry of Resin Formulation B (described above). A stencil was used to create the repeating dot pattern. The stencil had holes that were 2.5 mm in diameter with a center-to-center spacing of 7.5 mm in offset rows. The resulting printed web was passed through a heating apparatus (set at 1.7 meters per minute and 160° C) to solidify the dots of resin.

The nonwoven web inner layer was prepared using a conventional air-laying web forming machine (available from the Rando Machine Company, Macedon, NY under the trade designation RANDO WEBBER). The web was a blend of viscose fibers (52% by weight), PET fibers (18% by weight), and melty fibers (30% by weight). The viscose fiber (diameter of 10-18 microns) was obtained as LENZING viscose fiber from the Lenzing Group (Lenzing, Austria) and had a fiber length of 3.9 cm. The PET fiber (3 denier) was obtained from the INVISTA Company (Wichita, KS) and had a fiber length of 3.8 cm. The melty fiber (4 denier) was Tairilin polyester Type LML41 (NanYa Plastics Corporation, Kaohsiung City, Taiwan) and had a fiber length of 5 cm. The resulting nonwoven web had a nominal basis weight of 222 grams per square meter (gsm) and a web thickness of 5.2 mm. The solids content of the nonwoven web was about 2.9%. The web was needle tacked and thermally bonded by passing the web through a heating apparatus (set at 1.1 meters per minute and 138° C).

Each nonwoven layer was cut into 1.5 inch by 3 inch (3.8 cm by 7.6 cm) sections. The layers were stacked and edge aligned so that one surface of the inner nonwoven layer was in contact with the first outer layer and the opposite surface of the inner layer was in contact with the second outer layer. The second outer layer was oriented so that the scouring layer was exposed (i.e. facing away from the inner layer). The edges were heat sealed at 232° C to form the finished sampling article.

### Example 3. Testing of Articles for Recovery of a Bacterial Sample (cfu) from a Surface

A 10mL bacterial suspension containing a mixture of *Escherichia coli* (ATCC 35150) and *Salmonella typhimurium* (ATCC 51812) was prepared using an overnight culture of each strain propagated in 5 mL of Tryptic soy broth (from Thermo Fisher Scientific, Waltham, MA) at 35 °C for 24 hours. The suspension was serially diluted with Butterfields's buffer (from the 3M Corporation, St. Paul, MN) to a target concentration of about 500 cfu/mL (colony forming units/mL) .

Stainless steel coupons (10.2 cm by 10.2 cm) were individually placed in glass Petri dishes and sterilized in an autoclave for 15 minutes at 121 °C. The entire surface on one side of each coupon was coated with 1 mL of the bacterial suspension using a FISHERBRAND L-shaped cell spreader (from Thermo Fisher Scientific). The coated stainless steel coupons were allowed to air dry under ambient conditions until no visible inoculum could be seen. The dried coupons were tested for bacterial removal using either the finished Article of Example 1; a dry, biocide-free, gamma irradiated, cellulose sponge (3.8 cm by 7.6 cm) obtained from the 3M Corporation (product #BP133ES) (Comparative Article A); or a polyurethane foam sponge (3.8 cm by 7.6 cm) cut from a polyurethane foam sheet obtained from Woodbridge INAOC Technical Products, Chattanooga, TN (Comparative Article B). The polyurethane sponges were sterilized using ethylene oxide (37 °C, 60% relative humidity, 3 hour exposure). The tests were conducted in triplicate using a single sampling article for each coupon (Test Coupons #1-3). Each sampling article was hydrated with 10 mL of sterile Butterfield's buffer (from the 3M Corporation). The coated surface of each coupon was sampled using a hydrated article according to the procedure described in section 7.5.4.2 of ISO18593:2018 Standard `Microbiology of the Food Chain - Horizontal Methods for Surface Sampling'(International Organization for Standardization, Geneva, Switzerland).

For the coupons tested with an Article of Example 1, each coupon was first gently scrubbed with the surface of the article containing the scouring layer and then wiped for the collection of the bacterial analyte using the opposite, non-scouring surface (i.e. nonwoven first layer) of the article. Following the sampling of the coupons, each article was sealed in a separate polyethylene/polyester laminated (gamma irradiated) sample bag (12.7 cm by 22.9 cm, product #SSL100, from the 3M Corporation) and stored in a refrigerator (2-8 °C) for 96 hours prior to enumeration.

Enumeration was conducted by the following procedure. The outside of each bag was squeezed by hand for two minutes in a manner so as to release the liquid from the article into the bag. Two 1 mL samples of liquid (Sample 1 and Sample 2) were removed from each bag using a pipette and individually plated onto separate 3M PETRIFILM Rapid Aerobic Count (PRAC) plates (from the 3M Corporation) according to the manufacturer's instructions. The inoculated plates were incubated at 35 °C for 24 hours. After the incubation period, the total colony count (cfu) for each plate was determined using a 3M PETRIFILM Plate Reader (from the 3M Corporation). The results are reported in Table 3.

**Table 3.**

| | Total Colony Count (cfu) | | | | | |
|---|---|---|---|---|---|---|
| | Test Coupon #1 | | Test Coupon #2 | | Test Coupon #3 | |
| | Sample 1 | Sample 2 | Sample 1 | Sample 2 | Sample 1 | Sample 2 |
| Article of Example 1 | 180 | 164 | 211 | 217 | 90 | 97 |
| Comparative Article A | 24 | 31 | 20 | 16 | 8 | 6 |
| Comparative Article B | 64 | 58 | 7 | 11 | 28 | 34 |

### Example 4. Testing of Articles for Release of Bacterial Sample (cfu) from an Article

A bacterial suspension of *Escherichia coli* (ATCC 35150) and *Salmonella typhimurium* (ATCC 51812) was prepared as described in Example 3. Sterile NASCO WHIRL-PAK sample bags (product #01-812-1A, from Thermo Fisher Scientific) were individually assembled with each bag containing a single sampling article selected from either the Article of Example 1, Comparative Article 1, or Comparative Article 2. Each sampling article was hydrated with 10 mL of sterile Butterfield's buffer (from the 3M Corporation) and then inoculated with 1 mL of the bacterial suspension (target concentration of 500 cfu/mL). Following inoculation, each bag was sealed with the corresponding sampling article inside and stored in a refrigerator (2-8 °C) for 96 hours. Bags containing each type of sampling article were prepared in triplicate.

Each bag was then removed from the refrigerator and the outside of each bag was squeezed by hand for two minutes in a manner so as to release the liquid from the article into the bag. Two 1 mL samples of liquid (Sample 1 and Sample 2) were removed from the bag using a pipette and individually plated onto separate 3M PETRIFILM Rapid Aerobic Count (PRAC) plates (from the 3M Corporation) according to the manufacturer's instructions. The inoculated plates were incubated at 35 °C for 24 hours. After the incubation period, the total colony count (cfu) for each plate was determined using a 3M PETRIFILM Plate Reader (from the 3M Corporation). The results are reported in Table 4.

**Table 4.**

| | Total Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 226 | 209 | 395 | 354 | 172 | 155 |
| Comparative Article A | 8 | 21 | 29 | 25 | 68 | 66 |
| Comparative Article B | 121 | 117 | 67 | 72 | 26 | 31 |

### Example 5. Testing of Articles for Release of Bacterial Sample (cfu) using Escherichia coli as the Inoculum

A 10 mL bacterial suspension containing a mixture of *Escherichia coli* (ATCC 35150) was prepared using an overnight culture propagated in 5ml of tryptic soy broth (from Thermo Fisher Scientific) at 35 °C for 24 hours. The suspension was serially diluted with Butterfields's buffer (from the 3M Corporation) to a target concentration of about 30-100 cfu/mL.

Sterile WHIRL-PAK sample bags (product #01-812-1A, from Thermo Fisher Scientific) were individually assembled containing a single sampling article selected from either the Article of Example 1, the Article of Example 2, or a negative control article. Articles of Example 1 and Example 2 were hydrated with 10 mL of sterile Butterfield's buffer (from the 3M Corporation,) followed by inoculation with 1 mL of the E. *coli* suspension. Negative control articles were prepared by omitting the addition of E. *coli* suspension to hydrated articles of Examples 1 and 2. Next, each bag was sealed with the corresponding sampling article inside. The bags were separated into 2 sets of 9 bags (3 bags for each type of sample article in a set).

The articles of the first set of bags were immediately tested (Test Time = 0 hours) following inoculation. Each bag in the first set was squeezed by hand for two minutes in a manner so as to release the liquid from the article into the bag. Two 1 mL samples of liquid (Sample 1 and Sample 2) were removed from each bag using a pipette and individually plated onto separate 3M PETRIFILM Rapid Aerobic Count (PRAC) plates (from the 3M Corporation) according to the manufacturer's instructions. The inoculated plates were incubated at 35 °C for 24 hours. After the incubation period, the total colony count (cfu) for each plate was determined using a 3M PETRIFILM Plate Reader (from the 3M Corporation).

The bags of the second set were stored in a refrigerator (2-8 °C) for 96 hours following the inoculation step. The bags were removed from the refrigerator and tested (Test Time = 96 hours) using the same procedure as described for the first set. The results are reported in Tables 5 and 6. No colony growth (cfu count) was observed for the negative control articles.

**Table 5. E. coli (1^{st} Set): Test Time = 0 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 21 | 21 | 23 | 18 | 32 | 25 |
| Article of Example 2 | 28 | 24 | 38 | 31 | 31 | 30 |

**Table 6. E. coli (2nd Set): Test Time = 96 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 18 | 20 | 29 | 27 | 30 | 23 |
| Article of Example 2 | 32 | 21 | 35 | 29 | 30 | 34 |

### Example 6. Testing of Articles for Release of Bacterial Sample (cfu) using Salmonella typhimurium as the Inoculum

The same procedure as described in Example 5 was followed with the exception that a suspension of *Salmonella typhimurium* (ATCC 51812) was used as the inoculum (1 mL with a target concentration of about 30-100 cfu/mL), instead of the E. *coli* suspension. The results are reported in Tables 7 and 8. No colony growth (cfu count) was observed for the negative control articles.

**Table 7. S typhimurium (1st Set): Test Time = 0 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 28 | 30 | 33 | 32 | 31 | 38 |
| Article of Example 2 | 52 | 52 | 64 | 52 | 60 | 61 |

**Table 8. S typhimurium (2nd Set): Test Time = 96 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 22 | 23 | 29 | 25 | 32 | 27 |
| Article of Example 2 | 52 | 60 | 59 | 45 | 58 | 62 |

### Example 7. Testing of Articles for Release of Bacterial Sample (cfu) using Listeria monocytogenes as the Inoculum

The same procedure as described in Example 5 was followed with the exception that a suspension of *Listeria monocytogenes* (ATCC 7644) was used as the inoculum (1 mL with a target concentration of about 30-100 cfu/mL), instead of the E. *coli* suspension. The results are reported in Tables 9 and 10. No colony growth (cfu count) was observed for the negative control articles.

**Table 9. L monocytogenes (1^{st} Set): Test Time = 0 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 62 | 70 | 45 | 58 | 51 | 60 |
| Article of Example 2 | 80 | 60 | 62 | 78 | 58 | 70 |

**Table 10. L monocytogenes (2nd Set): Test Time = 96 hours**

| | Colony Count (cfu) | | | | | |
|---|---|---|---|---|---|---|
| | Test #1 | | Test #2 | | Test #3 | |
| | Sample 1 | Sample 2 | Sample 1 | Sample 2 | Sample 1 | Sample 2 |
| Article of Example 1 | 45 | 59 | 40 | 49 | 44 | 53 |
| Article of Example 2 | 70 | 55 | 53 | 65 | 53 | 51 |

## Claims

1. An article comprising an inner layer contained within one or more outer layers, the inner layer comprising a hydrophilic, absorbent porous textile having a solids content of 0.5%-30%, optionally 0.5% to 15%; and
the one or more outer layers comprising a porous, hydrophilic, wicking textile having a solids content of about 2%-40%; and
further comprising:
a scouring layer disposed over a portion of at least one of the one or more outer layers, and
one or more second portions of the one or more outer layers, wherein the scouring layer is not disposed over the one or more second portions.

2. The article of any of the preceding claims, wherein the scouring layer comprises a cured resin and optionally further comprises a plurality of abrasive particles having a mohs hardness of 3 or greater.

3. The article of any of the preceding claims, wherein the scouring layer comprises a plurality of abrasive particles, and wherein the plurality of abrasive particles comprises glass particles, ceramic particles, or a combination thereof.

4. The article of any of the preceding claims, wherein the hydrophilic, absorbent porous textile of the inner layer comprises viscose fibers, rayon fibers, or both viscose and rayon fibers.

5. The article of any of the preceding claims, wherein the porous, hydrophilic, wicking textile of the one or more outer layers comprises viscose fibers, rayon fibers, or both viscose and rayon fibers.

6. The article of any of claims 4 or 5, wherein at least part of the viscose fibers or rayon fibers thermally bonded to poly(ethylene terephthalate).

7. The article of any of the preceding claims, wherein either the article comprises a first outer layer and a second outer layer, wherein
the first outer layer is sealed to the second outer layer at one or more seals; and
the inner layer is disposed between the first outer layer and the second outer layer; or the article comprises a single outer layer, wherein
the single outer layer is sealed to itself at one or more seals; and
wherein the inner layer is contained within the single outer layer.

8. The article of any of the preceding claims wherein the scouring layer is disposed at least a first portion of the one or more outer layers in the form of a plurality of lines or geometrical shapes over more than one first portions of the one or more outer layers, the plurality of lines or geometrical shapes being present in a repeating pattern on the one or more outer layers.

9. The article of any of the preceding claims, further comprising a handle.

10. The article of any of the preceding claims, wherein the inner layer comprises a hydrophilic, absorbent porous textile that is selected from a nonwoven, a woven, and a knit; and/or the one or more outer layers comprises a porous, hydrophilic, wicking textile that is selected from a nonwoven, a woven, and a knit.

11. The article of any of the preceding claims, wherein the hydrophilic, absorbent porous textile is a nonwoven, and/or the porous, hydrophilic, wicking textile is a nonwoven.

12. The article of any of the preceding claims, wherein the inner layer and the one or more outer layers are biocide free.

13. A method of collecting a sample of microorganisms from a surface, the method comprising contacting a surface with the article of any of the preceding claims.

14. The method of claim 13, wherein the surface is a food preparation surface, a workstation surface, or a machine surface; or wherein the surface is a biological surface.

15. The method of claim 13 or14, wherein the surface is a biological surface, and the biological surface is a biofilm-coated surface, a skin surface, or a wound surface.

## Patentansprüche

1. Ein Artikel, der eine innere Schicht beinhaltet, die im Innern von einer oder mehreren äußeren Schichten enthalten ist, wobei die innere Schicht eine hydrophile, saugfähige poröse Textilie beinhaltet, die einen Feststoffanteil von 0,5%-30%, optional 0,5% bis 15% aufweist; und wobei die eine oder mehreren äußeren Schichten eine poröse, hydrophile, feuchtigkeitsabführende Textilie beinhalten, die einen Feststoffanteil von etwa 2%-40% aufweist; und
der ferner folgendes beinhaltet:
eine scheuernde Schicht, die über einem Abschnitt von mindestens einer von der einen oder den mehreren äußeren Schichten angeordnet ist, und
einen oder mehrere zweite Abschnitte der einen oder mehreren äußeren Schichten, wobei die scheuernde Schicht nicht über dem einen oder den mehreren zweiten Abschnitten angeordnet ist.

2. Artikel nach Anspruch 1, wobei die scheuernde Schicht ein gehärtetes Harz beinhaltet und optional ferner eine Vielzahl von abreibenden Teilchen mit einer Mohshärte von 3 oder mehr beinhaltet.

3. Artikel nach einem der vorhergehenden Ansprüche, wobei die scheuernde Schicht eine Vielzahl von abreibenden Teilchen beinhaltet, und wobei die Vielzahl von abreibenden Teilchen Glasteilchen, Keramikteilchen oder eine Kombination davon beinhaltet.

4. Artikel nach einem der vorhergehenden Ansprüche, wobei die hydrophile, saugfähige poröse Textilie der inneren Schicht Viskosefasern, Rayonfasern oder sowohl Viskose- als auch Rayonfasern beinhaltet.

5. Artikel nach einem der vorhergehenden Ansprüche, wobei die poröse, hydrophile, feuchtigkeitsabführende Textilie der einen oder mehreren äußeren Schichten Viskosefasern, Rayonfasern oder sowohl Viskose- als auch Rayonfasern beinhaltet.

6. Artikel nach einem der Ansprüche 4 oder 5, wobei mindestens ein Teil der Viskosefasern oder Rayonfasern thermisch mit Poly(ethylenterephthalat) gebunden ist.

7. Artikel nach einem der vorhergehenden Ansprüche, wobei entweder der Artikel eine erste äußere Schicht und eine zweite äußere Schicht beinhaltet, wobei
die erste äußere Schicht mit der zweiten äußeren Schicht an einer oder mehreren Versiegelungen versiegelt ist; und
die innere Schicht zwischen der ersten äußeren Schicht und der zweiten äußeren Schicht angeordnet ist;
oder der Artikel eine einzelne äußere Schicht beinhaltet, wobei
die einzelne äußere Schicht an einer oder mehreren Versiegelungen mit sich selbst versiegelt ist; und
wobei die innere Schicht im Innern der einzelnen äußeren Schicht enthalten ist.

8. Artikel nach einem der vorhergehenden Ansprüche, wobei die scheuernde Schicht mindestens auf einem ersten Abschnitt der einen oder mehreren äußeren Schichten in Form einer Vielzahl von Linien oder geometrischen Gestalten über mehr als einem ersten Abschnitt der einen oder mehreren äußeren Schichten angeordnet ist, wobei die Vielzahl von Linien oder geometrischen Gestalten in einem wiederholenden Muster auf der einen oder den mehreren äußeren Schichten vorhanden ist.

9. Artikel nach einem der vorhergehenden Ansprüche, der ferner einen Handgriff beinhaltet.

10. Artikel nach einem der vorhergehenden Ansprüche, wobei die innere Schicht eine hydrophile, saugfähige poröse Textilie beinhaltet, die aus einem Vlies, einem Gewebe und einem Gestrick ausgewählt ist; und/oder wobei die eine oder mehreren äußeren Schichten eine poröse, hydrophile, feuchtigkeitsabführende Textilie beinhalten, die aus einem Vlies, einem Gewebe und einem Gestrick ausgewählt ist.

11. Artikel nach einem der vorhergehenden Ansprüche, wobei die hydrophile, saugfähige poröse Textilie ein Vlies ist und/oder die poröse, hydrophile, feuchtigkeitsabführende Textilie ein Vlies ist.

12. Artikel nach einem der vorhergehenden Ansprüche, wobei die innere Schicht und die eine oder mehreren äußeren Schichten biozidfrei sind.

13. Ein Verfahren zur Entnahme einer Probe von Mikroorganismen von einer Oberfläche, wobei das Verfahren das In-Kontakt-Bringen einer Oberfläche mit dem Artikel nach einem der vorhergehenden Ansprüche beinhaltet.

14. Verfahren nach Anspruch 13, wobei die Oberfläche eine Nahrungsmittelzubereitungsoberfläche, eine Arbeitsplatzoberfläche oder eine Maschinenoberfläche ist; oder wobei die Oberfläche eine biologische Oberfläche ist.

15. Verfahren nach Anspruch 13 oder 14, wobei die Oberfläche eine biologische Oberfläche ist und die biologische Oberfläche eine mit Biofilm beschichtete Oberfläche, eine Hautoberfläche oder eine Wundoberfläche ist.

## Revendications

1. Article comprenant une couche interne contenue dans une ou plusieurs couches externes, la couche interne comprenant un textile poreux absorbant hydrophile ayant une teneur en solides de 0,5 % à 30 %, optionnellement de 0,5 % à 15 % ; et
les une ou plusieurs couches externes comprenant un textile à effet de mèche hydrophile poreux ayant une teneur en solides d'environ 2 % à 40 % ; et
comprenant en outre :
une couche de décapage disposée par-dessus une partie d'au moins une des une ou plusieurs couches externes, et
une ou plusieurs deuxièmes parties des une ou plusieurs couches externes, la couche de décapage n'étant pas disposée par-dessus les une ou plusieurs deuxièmes parties.

2. Article selon la revendication 1, dans lequel la couche de décapage comprend une résine durcie et comprend optionnellement en outre une pluralité de particules abrasives ayant une dureté Mohs de 3 ou plus.

3. Article selon l'une quelconque des revendications précédentes, dans lequel la couche de décapage comprend une pluralité de particules abrasives, et dans lequel la pluralité de particules abrasives comprend des particules de verre, des particules de céramique, ou une combinaison de celles-ci.

4. Article selon l'une quelconque des revendications précédentes, dans lequel le textile poreux absorbant hydrophile de la couche interne comprend des fibres de viscose, des fibres de rayonne, ou à la fois des fibres de viscose et de rayonne.

5. Article selon l'une quelconque des revendications précédentes, dans lequel le textile à effet de mèche hydrophile poreux des une ou plusieurs couches externes comprend des fibres de viscose, des fibres de rayonne, ou à la fois des fibres de viscose et de rayonne.

6. Article selon l'une quelconque des revendications 4 ou 5, dans lequel au moins une partie des fibres de viscose ou des fibres de rayonne sont thermoliées à du poly(téréphtalate d'éthylène).

7. Article selon l'une quelconque des revendications précédentes, dans lequel soit l'article comprend une première couche externe et une deuxième couche externe, dans lequel
la première couche externe est appliquée hermétiquement sur la deuxième couche externe au niveau d'un ou plusieurs joints d'étanchéité ; et
la couche interne est disposée entre la première couche externe et la deuxième couche externe ;
soit l'article comprend une couche externe unique, dans lequel
la couche externe unique est appliquée hermétiquement sur elle-même au niveau d'un ou plusieurs joints d'étanchéité ; et
dans lequel la couche interne est contenue dans la couche externe unique.

8. Article selon l'une quelconque des revendications précédentes, dans lequel la couche de décapage est disposée sur au moins une première partie des une ou plusieurs couches externes sous la forme d'une pluralité de lignes ou de formes géométriques par-dessus plus d'une première partie des une ou plusieurs couches externes, la pluralité de lignes ou de formes géométriques étant présente dans un motif répétitif sur les une ou plusieurs couches externes.

9. Article selon l'une quelconque des revendications précédentes, comprenant en outre une poignée.

10. Article selon l'une quelconque des revendications précédentes, dans lequel la couche interne comprend un textile poreux absorbant hydrophile qui est sélectionné parmi un non-tissé, un tissé et un tricot ; et/ou les une ou plusieurs couches externes comprennent un textile à effet de mèche hydrophile poreux qui est sélectionné parmi un non-tissé, un tissé et un tricot.

11. Article selon l'une quelconque des revendications précédentes, dans lequel le textile poreux absorbant hydrophile est un non-tissé et/ou le textile à effet de mèche hydrophile poreux est un non-tissé.

12. Article selon l'une quelconque des revendications précédentes, dans lequel la couche interne et les une ou plusieurs couches externes sont exemptes de biocides.

13. Procédé de prélèvement d'un échantillon de micro-organismes sur une surface, le procédé comprenant la mise en contact d'une surface avec l'article selon l'une quelconque des revendications précédentes.

14. Procédé selon la revendication 13, dans lequel la surface est une surface de préparation d'aliments, une surface de poste de travail, ou une surface de machine ; ou dans lequel la surface est une surface biologique.

15. Procédé selon la revendication 13 ou 14, dans lequel la surface est une surface biologique, et la surface biologique est une surface recouverte d'un biofilm, une surface de peau, ou une surface de plaie.
